# EUROPÄISCHE PATENTSCHRIFT

(11) **EP 2 046 713 B1**
(45) Veröffentlichungstag und Bekanntmachung des Hinweises auf die Patenterteilung: **17.10.2018**
(21) Anmeldenummer: 07786227.4
(22) Anmeldetag: 20.07.2007
(51) Int. Cl.: B01J 8/00, C07C 51/235, C07C 51/25, B01J 8/06

(54) **VORRICHTUNG UND VERFAHREN ZUR HERSTELLUNG VON ACRYLSÄURE MIT VERMINDERTER AUTOXIDATIONSNEIGUNG**
DEVICE AND METHOD FOR PRODUCING ACRYLIC ACID WITH A REDUCED AUTOXIDATION TENDENCY
DISPOSITIF ET PROCÉDÉ POUR LA PRÉPARATION D'ACIDE ACRYLIQUE AVEC TENDANCE RÉDUITE À L'AUTO-OXYDATION

(30) Priorität: 21.07.2006 DE 102006036177
(43) Veröffentlichungstag der Anmeldung: 15.04.2009
(73) Patentinhaber: Evonik Degussa GmbH, 45128 Essen (DE)
(72) Erfinder: MOSLER, Jürgen, 44577 Castrop-Rauxel (DE); BUB, Günther, 45772 Marl (DE)
(74) Vertreter: Lang, Arne
(86) Internationale Anmeldenummer: PCT/EP2007/006479
(87) Internationale Veröffentlichungsnummer: WO 2008/009466

(56) Entgegenhaltungen:
- EP-A- 0 834 518
- WO-A-99/41011
- WO-A-2004/108267
- WO-A1-01/45758
- DE-A1- 19 829 477
- DE-A1- 19 922 156
- DE-A1-102004 008 574
- US-B1- 6 352 619

## Beschreibung

Gegenstand der vorliegenden Erfindung ist eine Vorrichtung und ein Verfahren zur Herstellung von Acrylsäure.

Ausgangsstoff für eine Vielzahl von Polymeren ist Acrylsäure. Acrylsäure ist insbesondere auch Ausgangsstoff für so genannte superabsorbierende Polymere, welche auf vernetzten, teilneutralisierten Polyacrylaten basieren und mehr als das zehnfache ihres Eigengewichts an Wasser aufnehmen können. Acrylsäure wird regelmäßig aus Acrolein hergestellt, welches wiederum durch Gasphasenoxidation von Propen erzeugt wird. Bei dieser Gasphasenoxidation von Propen stellt die so genannten Autoxidation oftmals ein Problem dar, bei dem statt einer gewünschten partiellen Oxidation des Propens unter Bildung von Acrolein eine unerwünschte vollständige Oxidation des Propens unter Bildung unerwünschter Nebenprodukte erfolgt.

Die Gasphasenoxidation von Propen über Acrolein zu Acrylsäure ist unter anderem in WO 2004/108267 A1 und WO 99/41011 A1 beschreiben.

Genauer gesagt, beschreibt WO 2004/108267 A1 ein Verfahren zur thermischen Behandlung der Vorläufermasse einer katalytischen Aktivmasse in einem von einem Gasstrom durchströmten Drehrohrofen, wobei wenigsten eine Teilmenge des den Drehrohrofen durch-strömenden Gasstroms im Kreis geführt wird.

Die WO 99/41011 A1 offenbart ein Reaktionsrohr bzw. ein Rohrbündelreaktor mit Reaktionsrohr zur Durchführung einer heterogen katalysierten Gasphasenoxidation in der das Auftreten von "hot spots" überwiegend oder vollständig vermieden wird in dem das Reaktionsrohr eine katalytische Beschichtung aufweist, die aus einer Multimetalloxidmasse besteht.

EP 834 518 A1 offenbart ein Verfahren zur Herstellung von Homo - und Copolymeren aus der Gruppe Styrol, Butadien, Vinylchlorid, Vinylacetat, Vinylidenchlorid, Alkyl(meth)acrylat, (Meth)acrylsäure, (Meth)acrylnitril und (Meth)acrylnitril in Emulsionspolymerisationen bei einer Polymerisationstemperatur von mindstens 40°C in Anwesenheit eines Dispergierhilfsmittels und eines radikalischen Polymerisationsinitiators, wobei die Polymerisation mittels der Stufen; Wasser als inertes Lösemittel, gegebenenfalls Dispergierhilfsmittel, gegebenenfalls Saat und gegebenenfalls eine erste Teilmenge an Monomeren vorgegeben wird, in einer zweiten Stufe Initiator, und in einer dritten Stufe die Restmenge oder die vollständige Menge an Monomeren direkt oder in Emulsionsform in Anwesenheit von weiterem Wasser und gegebenenfalls weiteren Dispergiermittel oder anderen Hilfsstoffen zugesetzt werden, wobei die Stufen 1 und 2 oder 2 und 3 jeweils auch einstufig gefahren werden können, und in einzelnen oder allen Stufen das als Dispersion vorliegenden Reaktionsgemisch durch einen vom und wieder zum Reaktionsgefäß führenden externen Kreislauf bewegt wird, der mindestens eine scherungsarme Pumpe und mindestens einen Wärmetauscher mit einem im wesentlichen laminaren Strömungsprofil aufweist, und die Polymerisationstemperatur zwischen 40°C und 120°C liegt. Diese Schrift erwähnt auch die Wichtigkeit von laminaren Strömungsprofilen. Der laminare Strom bezieht sich dabei auf eine Emulsion für eine Polymerisation und trifft nur auf den Wärmetauscher zu.

DE 10 2004 008574 A1 offenbart ein Verfahren zur Reinigung von (Meth)acrylsäure aus destillativer Aufarbeitung mittels Dimerenspaltung, wobei Verunreinigungen wie Maleinsäure, Maleinsäureanhydrid und Protoanemonin gezielt abgetrennt werden. Es handelt sich hierbei um einen nachgeordneten Schritt der (Meth)acrylsäureherstellung, der die Ausbeuterhöhung der (Meth)acrylsäure, durch die Aufarbeitung von höheren Folgeprodukten, betrifft. Diese Schrift erwähnt auch Polymere auf Basis von Acrylsäure und deren Weiterverarbeitung und Verwendung.

US 6 352 619 offenbart ein Verfahren zur Reinigung von Acrylsäure, mit dem die Verunreinigungen vom Polymertyp an den Stellen der Destillationskolonnen beseitigt werden sollen, an denen sie sich bevorzugt anreichern, und mit dem insbesondere die Polymerverunreinigung beseitigt werden sollen, wobei die Destillationskolonne der zu reinigenden Acrylsäure in Gegenwart mindestens einer Verbindung aus der Gruppe der nichtionischen grenzflächenaktiven Stoffe durchgeführt wird.

DE 19922156 A1 offenbart ein Verfahren zur Herstellung von Multimetalloxidmassen und deren Verwendung.

US 2008/045748 offenbart eine Apparatur, die mindestens einen Verdränger aufweist, der den zur Verfügung stehenden Raum unterhalb eines Rohrbündels (einen Sammelraum) begrenzen soll, um die Verweilzeit des aus den Rohrbündeln austretenden Gases zu begrenzen.

DE 19829477 A1 beschreibt die Reinigung von Acrylsäure durch eine Kombination on Kristallisation und Destillation.

WO 0145758 A1 beschreibt die Herstellung eines Superabsorbers aus Acrylsäure.

Allgemein lag der vorliegenden Erfindung die Aufgabe zugrunde, die sich aus dem Stand der Technik ergebenden Nachteile im Zusammenhang mit der Herstellung von Acrylsäure durch zweistufige Gasphasenoxidation von Propen ergebenden Nachteile zu überwinden.

Insbesondere lag der vorliegenden Erfindung die Aufgabe zu Grunde, ein Verfahren und eine Vorrichtung zur Herstellung von Acrylsäure aus Propen anzugeben, mit dem bzw. mit der die Acrylsäure in möglichst hoher Ausbeute erhalten werden kann.

Insbesondere lag der vorliegenden Erfindung die Aufgabe zugrunde, eine Vorrichtung und ein Verfahren zur Herstellung von Acrylsäure vorzuschlagen, bei dem die Autoxidation von Acrolein im Vergleich zu herkömmlichen Reaktoren bzw. herkömmlichen Verfahren verringert wird.

Einen Beitrag zur Lösung der vorstehenden Aufgaben leisten die Gegenstände der Ansprüche, wobei die abhängigen Ansprüche besondere Ausführungsformen der Erfindung darstellen.

Die erfindungsgemäße Vorrichtung zur Herstellung von Acrylsäure umfasst
- einen ersten Reaktor und
- mindestens einen weiteren Reaktor,
   -- wobei zumindest der erste Reaktor ein Rohrbündelreaktor mit einer Mehrzahl von Röhren ist, die einen Katalysator umfassen, der die Synthese von Acrolein aus Propen katalysiert,
   -- wobei die Röhren in einen Sammelraum münden, der über einen Abströmbereich mit dem mindestens einen weiteren Reaktor fluidleitend verbindbar ist,
   -- wobei der Abströmbereich ein Laminarisierungsmittel umfasst, welches eine Laminarisierung eines Strömungsprofils eines durch den Abströmbereich strömenden Gases bewirkt und
   -- wobei das Laminarisierungsmittel in den Abströmbereich hineinragt.

Unter "fluidleitend" wird erfindungsgemäß verstanden, dass Gase oder Flüssigkeiten, Suspensionen eingeschlossen, oder deren Mischungen, vorzugsweise Gase, durch entsprechende Leitungen geführt werden. Hierzu lassen sich insbesondere Rohleitungen, Pumpen und der gleichen einsetzen.

In einer Ausgestaltung der vorliegenden Erfindung, können der erste und der zweite Reaktor Teile eines Großreaktors bilden, der den ersten und den darauf folgenden zweiten Reaktor aufnimmt. In einem solchen Großreaktor sind beide Reaktionsstufen mit jeweils unterschiedlichen Katalysatoren auf die Herstellung von Acrolein in dem ersten Teilreaktor und der Umsetzung von Acrolein zu Acrylsäure in dem zweiten Teilreaktor ausgelegt.

Unter einer "Laminarisierung eines Strömungsprofils" wird in diesem Zusammenhang insbesondere die Verringerung der Reynoldszahl der entsprechenden Strömung verstanden. Zur Bestimmung der Reynoldszahl sind dem Fachmann verschiedene Verfahren bekannt. Beispielsweise kann eine mittlere Reynoldszahl dadurch bestimmt werden, dass einerseits die Strömungsgeschwindigkeit bzw. der Massenstrom der Strömung bestimmt wird und andererseits die Viskosität des Gasstroms bestimmt wird. Die Strömungsgeschwindigkeit und/oder der Massenstrom können durch einen üblichen Durchflussmesser bestimmt werden, während die Viskosität des Gasstroms beispielsweise anhand der Zusammensetzung des Gasstroms und der Temperatur berechnet werden kann. Grundsätzlich kann auch eine Berechnung, insbesondere eine iterative Berechnung, anhand der entsprechenden Navier-Stokes-Gleichungen erfolgen. Insbesondere können hier übliche CFD-Methoden, beispielsweise das "FLUENT" Programmpaket, eingesetzt werden.

Weiterhin kann die Reynoldszahl durch bekannte Time-of-flight (TOF)- und/oder Markerverfahren bestimmt werden, in dem beispielsweise ein Strömungsprofil des Gases gemessen und eine Anpassung unter Variation der Reynoldszahl an dieses Profil erfolgt. Zudem kann die Laminarisierung ebenso vorteilhaft anhand eines Modells der Vorrichtung, beispielsweise anhand eines Reynoldschen Farbfadenversuchs, bestimmt werden.

Unter einem Rohrbündelreaktor versteht man insbesondere einen Reaktor, der eine Vielzahl von zueinander parallelen von Edukt- und Produktgasen durchströmbaren Röhren aufweist. Diese Röhren sind oft mit einer mindestens einen Katalysator umfassenden Schüttung gefüllt, wobei dieser so ausgewählt ist, dass eine entsprechende Reaktion katalysiert wird. Im Falle des ersten Reaktors ist der Katalysator insbesondere so ausgewählt, dass die Synthese von Acrolein aus Propen katalysiert wird. Dabei können als Katalysatoren alle dem Fachmann bekannten Katalysatoren enthalten sein, die üblicherweise bei der Gasphasenoxidation von Propen zu Acrolein eingesetzt werden. Insbesondere handelt es bei diesen Katalysatoren in der Regel um oxidische Mehrkomponentensysteme, welche üblicherweise auf Molybdän-, Chrom-, Vanadium- oder Telluroxiden basieren. Im Zusammenhang mit geeigneten Katalysatoren bei der Herstellung von Acrolein aus Propen wird auf "Stets Geforscht", Band 2, Chemieforschung im Degussa-Forschungszentrum Wolfgang 1988, S. 108-126, Kapitel "Acrolein und Derivate" Dietrich Arntz und Ewald Noll und insbesondere WO 03/051809 A1 verwiesen, wobei auf diesen Inhalt als Teil dieser Offenbarung Bezug genommen wird.

Besonders bevorzugt ist es, dass der im ersten Reaktor enthaltene Katalysator die Zusammensetzung

MoₐBi_{b}Fe_{c}A_{d}BₑC_{f}D_{g}Oₓ

aufweist, in der
- Mo: Molybdän,
- Bi: Wismut,
- Fe: Eisen,
- A: mindestens ein Element ausgewählt aus Kobalt und Nickel,
- B: mindestens ein Element, ausgewählt aus einem Alkalimetall, einem Erdalkalimetall und Thallium,
- C: mindestens ein Element, ausgewählt aus Wolfram, Silizium, Aluminium, Zirkonium und Titan,
- D: mindestens ein Element ausgewählt aus Phosphor, Tellur, Antimon, Zinn, Cer, Blei, Niob, Mangan, Arsen und Zink und
- O: Sauerstoff bedeuten
und in der, wenn a=12 ist,
- b: 0,1 bis 10,
- c: 0,1 bis 20,
- d: 2 bis 20,
- e: 0,001 bis 10,
- f: 0 bis 30,
- g: 0 bis 4 ist und x einen Wert hat, der durch den Oxidationszustand der anderen Elemente bestimmt ist.

Der Katalysator kann als solcher in die Röhren des Rohrbündelwärmeaustauschers eingebracht werden. Er kann aber auch auf inerte Katalysatorträger aufgebracht werden, die dann in die Röhren des Rohrbündelwärmeaustauschers eingebracht werden. Als Trägermaterialien können dabei übliche poröse oder unporöse Aluminiumoxide, Siliziumdioxid, Thoriumdioxid, Zirkondioxid, Siliziumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, zum Beispiel Kugeln oder Hohlzylinder, bevorzugt werden.

Im Allgemeinen sind Rohrbündelreaktoren so ausgebildet, dass eine Strömung eines Kühl- und/oder Temperiermittels in einer Durchströmungsrichtung senkrecht zur Durchströmungs- und Ausrichtung der Röhren durchgeführt werden kann. Als Kühl- oder Temperiermittel werden vorzugsweise fluide Medien eingesetzt. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

Gemäß der erfindungsgemäßen Vorrichtung ragt das Laminarisierungsmittel in den Abströmbereich hinein.

Durch das hineinragende Laminarisierungsmittel erfolgt eine Veränderung der Strömungsgeometrie im Abströmbereich. Besonders bevorzugt ist hierbei, dass sich das Laminarisierungsmittel in Richtung des Abströmbereichs verjüngt. Bevorzugt ist hierbei die Ausbildung eines Kegelstumpfes oder eines Kegels als Laminarisierungsmittel.

Gemäß einer weiteren vorteilhaften Ausgestaltung der erfindungsgemäßen Vorrichtung umfasst das Laminarisierungsmittel einen Kegelstumpf.

Gemäß einer weiteren vorteilhaften Ausgestaltung der erfindungsgemäßen Vorrichtung umfasst das Laminarisierungsmittel einen Kegel.

Insbesondere die Ausbildung eines Kegels oder Kegelstumpfes als Laminarisierungsmittel führt zu einer deutlichen Laminarisierung der Strömung, die wiederum zu geringeren Turbulenzraten und insbesondere zu einer deutlich kürzeren Verweilzeit der Edukte und Produkte in den einzelnen Röhren führt. Hierdurch wird in wirkungsvoller Weise das Risiko einer Autoxidation des Acroleins vermieden. Insbesondere kann so auch eine Schädigung des ersten Reaktors durch eine sich aufgrund der Autoxidation des Acroleins bildende Explosion verhindert werden.

Sofern es sich bei dem Laminarisierungsmittel um einen Kegel oder einen Kegelstumpf handelt, ist es bevorzugt, dass dieser Kegel oder Kegelstumpf einen Kegelwinkel in einem Bereich von 15 bis 60°, besonderes bevorzugt in einem Bereich von 20 bis 45° und darüber hinaus bevorzugt in einem Bereich von 25 bis 40° aufweist.

Unter einem Kegelwinkel wird hier insbesondere der halbe Öffnungswinkel des Kegels, d. h. der Winkel zwischen einer Projektion des Kegelstrumpfes bzw. des Kegels im Bereich des sich verjüngenden und gegebenenfalls spitzen Bereichs auf eine die Symmetrieachse des Kegels oder Kegelstumpfes umfassende Ebene verstanden.

Weiterhin ist es, wenn es bei sich dem Laminarisierungsmittel um einen Kegel oder einen Kegelstumpf handelt, bevorzugt, dass das Verhältnis von Höhe des Kegels bzw. Kegelstumpfes (H) : Durchmesser des Kegels bzw. Kegelstumpfes an der dem mindestens einen weiteren Reaktor abgewandten Seite (D) in einem Bereich von 3 : 1 bis 1 : 3, besonders bevorzugt in einem Bereich von 2 : 1 bis 1 : 2 und am meisten bevorzugt in einem Bereich von in einem Bereich von 1,5 : 1 bis 1 : 1,5 liegt.

Bei dem mindestens einen weiteren Reaktor handelt es sich vorzugsweise ebenfalls um einen Rohrbündelwärmeaustauscher. Denkbar ist jedoch auch ein Reaktor, in dessen Reaktionsraum Thermobleche derart angeordnet sind, dass sich zwischen den Thermoblechen Reaktionsräume und Wärmetransporträume ausbilden. Derartige Reaktoren sind beispielsweise in DE-A-198 48 208 oder DE-A-101 08 380 beschrieben.

Ebenso wie der erste Reaktor weist auch der mindestens eine weiteren Reaktor einen Katalysator auf, der, sofern es sich bei dem Reaktor um einen Rohrbündelwärmeaustauscher handelt, also solcher oder aber auf einem Katalysatorträgerkörper beschichtet in die Röhren des Rohrbündelwärmeaustauschers eingebracht sein kann. Sofern es sich bei dem mindestens einen weiteren Reaktor um einen Reaktor mit im Reaktionsraum angeordneten Thermoblechen handelt, kann der Katalysator als Festbettschüttung eingebracht oder aber auf der Oberfläche der Thermobleche beschichtet sein.

Bei dem Katalysator in dem mindestens einen weiteren Reaktor handelt es sich vorzugsweise um einen Katalysator, der die Umsetzung von Acrolein zu Acrylsäure katalysiert. Im Zusammenhang mit geeigneten Katalysatoren bei der Herstellung von Acrylsäure aus Acrolein wird wiederum auf "Stets Geforscht", Band 2, Chemieforschung im Degussa-Forschungszentrum Wolfgang 1988, S. 108-126, Kapitel "Acrolein und Derivate" Dietrich Arntz und Ewald Noll verwiesen.

Besonders bevorzugt ist es, dass der in dem mindestens einen weiteren Reaktor enthaltene Katalysator die Zusammensetzung

MOₐ V_{b}A_{c}B_{d}CₑD_{f}Oₓ

aufweist, in der
- Mo: Molybdän,
- V: Vanadium,
- A: mindestens ein Element ausgewählt aus Kupfer, Kobalt, Wismut und Eisen,
- B: mindestens ein Element, ausgewählt aus Antimon, Wolfram und Niob
- C: mindestens ein Element, ausgewählt aus Silizium, Aluminium, Zirkonium und Titan,
- D: mindestens ein Element ausgewählt aus einem Alkalimetall, einem Erdalkalimetall, Thallium, Phosphor, Tellur, Zinn, Cer, Blei, Mangan und Zink und
- O: Sauerstoff bedeuten
und in der, wenn a=12 ist,
- b: 0,1 bis 10,
- c: 0,1 bis 20,
- d: 0,1 bis 20,
- e: 0,001 bis 10,
- f: 0 bis 30 ist und x einen Wert hat, der durch den Oxidationszustand der anderen Elemente bestimmt ist.

Gemäß einem weiteren Aspekt der Erfindung wird ein Verfahren zur Herstellung von Acrylsäure vorgeschlagen, wobei
- in einer ersten Stufe Acrolein durch eine Gasphasenoxidation von Propen in einem ersten Reaktor erzeugt
- und in mindestens einer zweiten Stufe in mindestens einem weiteren Reaktor Acrolein in Acrylsäure umgesetzt wird,
   -- wobei der erste Reaktor ein Rohrbündelreaktor mit einer Mehrzahl von von Gas durchströmbaren Röhren ist, der über einen Abströmbereich mit mindestens einem weiteren Reaktor verbunden ist,
   -- wobei eine Gasströmung im Abströmbereich mittels eines Laminarisierungsmittels laminarisiert wird.

Das Verfahren ist erfindungsgemäß, wenn es in einer erfindungsgemäßen Vorrichtung durchgeführt wird.

Als Laminarisierungsmittel sind dabei diejenigen Laminarisierungsmittel gemeint, die bereits eingangs im Zusammenhang mit der erfindungsgemäßen Vorrichtung zur Herstellung von Acrylsäure genannt wurden.

Unter einer "Laminarisierung" wird auch hier insbesondere eine Verringerung der Reynoldszahl verstanden. Die Laminarisierung kann insbesondere durch eine entsprechende Ausbildung des Abströmbereichs erreicht werden. Insbesondere können im Abströmbereich Laminarisierungsmittel ausgebildet sein, die bevorzugt den Querschnitt, die Form und/oder die Länge des Abströmbereichs verändern. Insbesondere kann das erfindungsgemäße Verfahren an einer erfindungsgemäßen Vorrichtung durchgeführt werden.

Die Reynoldszahl kann insbesondere anhand einer Strömungsgeschwindigkeit und einer Viskosität des strömenden Gases bestimmt werden. Die dafür nötigen Größen können entweder gemessen werden, beispielsweise unter Verwendung eines Durchströmmessers, können andererseits auf Grund der bekannten Reaktionsbedingungen insbesondere unter Berücksichtigung der Temperatur, der Dichte der Gase, der Mischungsverhältnisse der Gase, des Zustandes der Katalysatoren, der Dichte der Katalysatoren, der Oberfläche, welche für eine katalytische Reaktion zur Verfügung steht, des Katalysators, der Strömungsquerschnitte und anderer Faktoren berechnet werden. Insbesondere können hier entsprechende Navier-Stokes-Gleichungen aufgestellt werden, die entsprechend gelöst werden. Bevorzugt können hier kommerzielle CFD (Continuous Fluid Dynamics)-Systeme zum Einsatzbekommen wie beispielsweise das "FLUENT"-Programmpaket. Die für die Vorrichtung zur Herstellung von Acrylsäure offenbarten Details und Vorteile sind in gleicher Weise auf das erfindungsgemäße Verfahren zur Herstellung von Acrylsäure anwend- und übertragbar und umgekehrt.

Vorzugsweise umfasst das Verfahren zur Herstellung von Acrylsäure als weiteren Verfahrensschritt neben der Erzeugung des Acroleins und der Umsetzung des Acroleins zur Acrylsäure auch die Aufreinigung der so erhaltenen Acrylsäure durch Destillation, Kristallisation, Extraktion oder durch eine Kombination dieser Aufreinigungsverfahren.

Vorzugsweise erfolgt die Aufreinigung dergestalt, dass zunächst das erhaltene Produktgasgemisch in einem sogenannten "Quenchtower" in Wasser unter Erhalt einer wässrigen Acrylsäurelösung einer Totalkondensation unterzogen wird. Denkbar ist jedoch auch, die Acrylsäure in hochsiedenden Lösungsmitteln, wie beispielsweise einem Gemisch aus 75 Gew.-% Diphenylether und 25 Gew.-% Diphenyl, zu absorbieren. Nach Absorption der Acrylsäure erfolgt dann üblicherweise eine weitere Aufreinigung durch Destillation, wobei im Falle wässriger Acrylsäurelösungen als Ausgangszusammensetzung häufig eine Azeotropdestillation in Gegenwart geeigneter Schleppmittel wie Toluol durchgeführt wird, wobei eine Roh-Acrylsäure als Sumpfprodukt zurückbehalten wird. Wurde das Reaktionsgasgemisch in hochsiedenden Lösungsmitteln absorbiert, so wird eine Roh-Acrylsäure üblicherweise im Seitenstrom einer Rektifikationsvorrichtung abgezogen.

Die so erhaltene Roh-Acrylsäure kann dann zur weiteren Aufreinigung weiter destilliert werden, um insbesondere Leichtsieder abzutrennen. Auch kann eine weitere Aufreinigung der Roh-Acrylsäure durch Kristallisation, insbesondere durch Suspensionskristallisation erfolgen, wobei letztendlich eine Rein-Acrylsäure mit einem Acrylsäuregehalt von mindestens 99 Gew.-% erhalten wird.

Des Weiteren wird ein Verfahren zur Herstellung eines hydrophilen Polymers offenbart, wobei die durch das hier beschriebene Verfahren erhältliche, vorzugsweise aufgereinigte Acrylsäure radikalisch polymerisiert wird. Vorzugsweise erfolgt die Polymerisation dergestalt, dass die durch das Verfahren erhältliche, vorzugsweise aufgereinigte Acrylsäure in teilneutralisierter Form in einer wässrigen Lösung in Gegenwart von Vernetzern radikalisch unter Bildung eines Hydrogels polymerisiert, das Hydrogel anschließend zerkleinert und getrocknet und die so erhaltenen Polymerpartikel anschließend oberflächenmodifiziert, vorzugsweise oberflächennachvernetzt werden. Auf diese Art und Weise werden sogenannte "Superabsorber" erhalten. Weitere Einzelheiten zu Superabsorbern, insbesondere zu deren Herstellung sind in *"*Modern Superabsorbent Polymer Technology", F. L. Buchholz, A. T. Graham, Wiley-VCH, 1998" offenbart.

Offenbart wird außerdem ein Verfahren zur Herstellung eines wasserabsorbierenden Hygieneartikels, bei dem ein hydrophiles, vorzugsweise wasserabsorbierendes Polymer, besonders bevorzugt ein Superabsorber, welches bzw. welcher nach dem vorstehenden Verfahren hergestellt wurde, in mindestens einem Hygieneartikelbestandteil eingearbeitet wird. Ein solcher Hygieneartikelbestandteil ist vorzugsweise ein Windel- oder Damenbinden-Core.

Offenbart werden auch chemische Produkte wie Fasern, Formkörper, Filme, Schäume, superabsorbierende Polymere, Detergentien, Spezialpolymere für die Bereiche Abwasserbehandlung, Dispersionsfarben, Kosmetika, Textilien, Lederveredlung oder Papierherstellung oder Hygieneartikel, die mindestens auf der aufgereinigten Acrylsäure basieren bzw. diese enthalten, wobei die aufgereinigte Acrylsäure nach dem vorgenannten Verfahren erhältlich ist.

Im Folgenden soll die Erfindung an Hand der beigefügten Figuren näher erläutert werden. Es zeigen:
- Fig. 1: schematisch eine erfindungsgemäße Vorrichtung im Schnitt;
- Fig. 2: schematisch einen Ausschnitt aus einer erfindungsgemäßen Vorrichtung und
- Fig. 3: schematisch ein Detail eines weiteren Ausführungsbeispiels einer erfindungsgemäßen Vorrichtung.

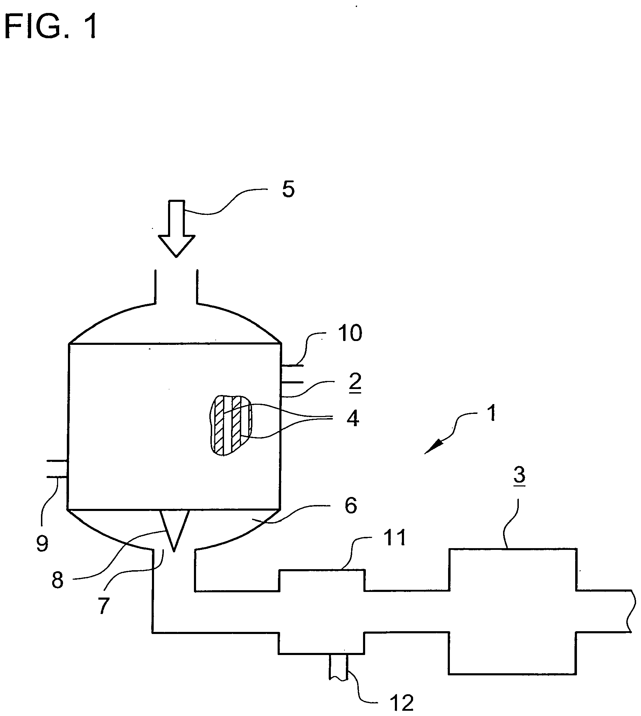

Fig. 1 zeigt schematisch eine erfindungsgemäße Vorrichtung 1 zur Herstellung von Acrylsäure. Die Vorrichtung 1 umfasst einen ersten Reaktor 2 und mindestens einen weiteren Reaktor 3. Zumindest der erste Reaktor 2 ist ein Rohrbündelreaktor mit einer Mehrzahl von Röhren 4, die der Übersichtlichkeit halber nur beispielhaft in einem Ausschnitt eingezeichnet sind. Die Röhren 4 können als Rohrbündelring angeordnet sein, um eine bessere Umströmung mit einem Kühlmedium, vorzugsweise einer Salzschmelze zu erreichen. Jede der Röhren 4 umfasst einen Katalysator zur katalytischen Umsetzung von Propen mit Sauerstoff zu Acrolein. Insbesondere kann dieser Katalysator ein Mehrkomponenten-Katalysator auf Basis von Wismutmolybdat sein. Die Röhren 4 können von einem ersten Gasstrom 5 durchströmt werden. Der erste Gasstrom 5 umfasst die Edukte der Gasphasenoxidation, also Propen und Sauerstoff. Das die Röhren 4 verlassende Gas wird in einem Sammelraum 6 zusammengeführt. Dieser Sammelraum 6 ist über einen Abströmbereich 7 mit dem mindestens einen weiteren Reaktor 3 verbunden. Der Abströmbereich 7 umfasst ein Laminarisierungsmittel 8, welches eine Laminarisierung eines Strömungsprofils eines durch den Abströmbereich 7 strömenden Gases bewirkt. Hierbei ragt das Laminarisierungsmittel 8 in den Abströmbereich 7 hinein und verjüngt sich in Richtung Abströmbereich 7. Sollten die Rohre 4 als Rohrbündelring angeordnet sein, ist es bevorzugt, das Laminarisierungsmittel 8 unter dem rohrfreien Innenbereich des Rohrbündelrings anzuordnen. Im vorliegenden ersten Ausführungsbeispiel ist das Laminarisierungsmittel 8 in Form eines Kegels ausgebildet. Unter einer Laminarisierung der Strömung wird eine Verminderung der Reynoldszahl dieser Strömung verstanden. Durch die Laminarisierung der Strömung kommt es zu einer Erhöhung der mittleren Strömungsgeschwindigkeit und zur Verminderung der Verweilzeit der Edukte und Produkte in den Röhren 4. Dies bewirkt, dass eine Autoxidation des Gasgemisches im Wesentlichen verhindert wird. Da die in den Röhren 4 ablaufenden Reaktionen auch exotherme Reaktionen umfassen, ist eine Temperierung der Röhren vorteilhaft. Hierzu ist ein Kühlmittelzulauf 9 und ein Kühlmittelablauf 10 ausgebildet, durch die ein Kühlmittel den ersten Reaktor 2 durch- und die Röhren 4 umströmen kann. Kühlmittelzulauf 9 und Kühlmittelablauf 10 können mit entsprechenden Mitteln zur Förderung eines Kühlmittels verbunden sein, beispielsweise einer entsprechenden nicht gezeigten Pumpe.

Über den Abströmbereich 7 verlässt das Produktgasgemisch den ersten Reaktor 2. In einem Mischer 11 erfolgt eine Mischung des Acroleins mit Sauerstoff, der über eine Zuleitung 12 zugebbar ist. Im weiteren Reaktor 3 erfolgt dann die Oxidation des Acroleins zu Acrylsäure.

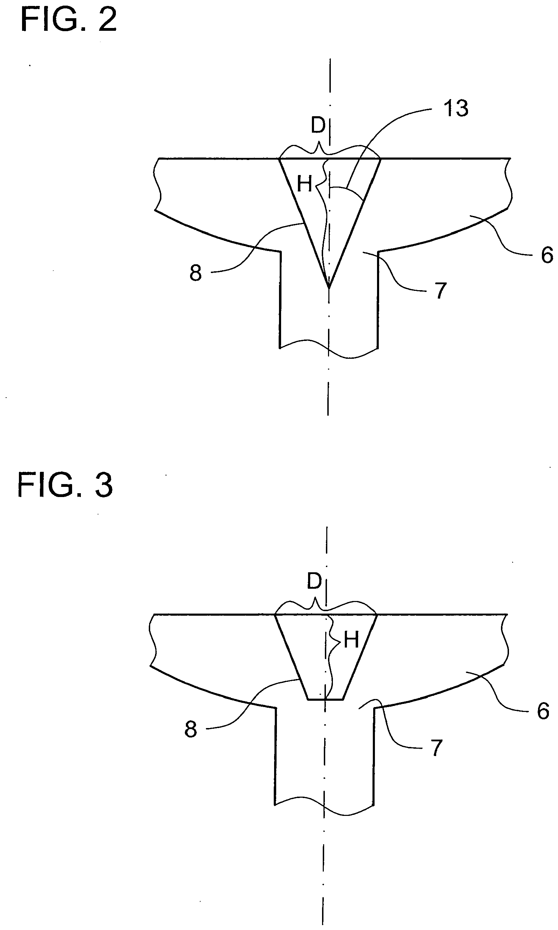

Fig. 2 zeigt schematisch einen Ausschnitt einer erfindungsgemäßen Vorrichtung 1. Gezeigt wird der Abströmbereich 7, der Sammelraum 6 und das Laminarisierungsmittel 8. Das Laminarisierungsmittel 8 umfasst einen Kegel mit einem Kegelwinkel 13, mit einer Höhe H und mit einem Durchmesser D an der dem mindestens einen weiteren Reaktor abgewandten Seite.

Fig. 3 zeigt keine erfindungsgemäße Ausführungsform, weil der Kegelstumpf (8) nicht in den Abströmbereich (7) hineinragt. Gezeigt sind auch hier der Sammelraum 6, der Abströmbereich 7 und das Laminarisierungsmittel 8. Das Laminarisierungsmittel 8 ist als Kegelstumpf ausgebildet.

### Bezugszeichenliste

- 1: Vorrichtung zur Herstellung von Acrylsäure
- 2: erster Reaktor
- 3: weiterer Reaktor
- 4: Röhre
- 5: erster Gasstrom
- 6: Sammelraum
- 7: Abströmbereich
- 8: Laminarisierungsmittel
- 9: Kühlmittelzulauf
- 10: Kühlmittelablauf
- 11: Mischer
- 12: Zuleitung
- 13: Kegelwinkel
- D: Durchmesser des Kegels bzw. des Kegelstumpfes an der dem mindestens einen weiteren Reaktor abgewandten Seite
- H: Höhe des Kegels bzw. des Kegelstumpfes

## Patentansprüche

1. Vorrichtung (1) zur Herstellung von Acrylsäure, umfassend
- einen ersten Reaktor (2) und
- mindestens einen weiteren Reaktor (3),
-- wobei zumindest der erste Reaktor (2) ein Rohrbündelreaktor mit einer Mehrzahl von Röhren (4) ist, die einen Katalysator umfassen, der die Synthese von Acrolein katalysiert,
-- wobei die Röhren (4) in einen Sammelraum (6) münden, der über einen Abströmbereich (7) mit dem mindestens einen weiteren Reaktor (3) fluidleitend verbindbar ist,
-- wobei der Abströmbereich (7) ein Laminarisierungsmittel (8) umfasst, welches eine Laminarisierung eines Strömungsprofils eines durch den Abströmbereich (8) strömenden Gases bewirkt und
-- wobei das Laminarisierungsmittel (8) in den Abströmbereich (7) hineinragt.

2. Vorrichtung (1) nach Anspruch 1, wobei das Laminarisierungsmittel (8) sich in Richtung des Abströmbereichs (7) verjüngt.

3. Vorrichtung (1) nach einem der vorhergehenden Ansprüche, wobei das Laminarisierungsmittel (8) einen Kegelstumpf umfasst.

4. Vorrichtung nach Anspruch 2 oder 3, wobei das Laminarisierungsmittel (8) einen Kegel umfasst.

5. Vorrichtung nach Anspruch 3 oder 4, wobei der Kegel oder Kegelstumpf einen Kegelwinkel (13) im Bereich von 15 bis 60° aufweist.

6. Vorrichtung nach Anspruch 5, wobei das Verhältnis von Höhe des Kegels bzw. Kegelstumpfes (H) : Durchmesser des Kegels bzw. Kegelstumpfes an der dem mindestens einen weiteren Reaktor abgewandten Seite (D) in einem Bereich von 3 : 1 bis 1 : 3 liegt.

7. Verfahren zur Herstellung von Acrylsäure, wobei
- in einer ersten Stufe Acrolein durch Gasphasenoxidation von Propen in einem ersten Reaktor (2) erzeugt
- und in mindestens einer zweiten Stufe in mindestens einem weiteren Reaktor (3) Acrolein in Acrylsäure umgesetzt wird,
-- wobei der erste Reaktor (2) ein Rohrbündelreaktor mit einer Mehrzahl von von Gas durchströmbaren Röhren (4) ist, der über einen Abströmbereich (7) mit mindestens einem weiteren Reaktor (3) verbunden ist,
-- wobei eine Gasströmung im Abströmbereich (7) mittels eines Laminisierungsmittels laminarisiert wird
wobei das Verfahren in einer Vorrichtung (1) nach einem der Ansprüche 1 bis 6 durchgeführt wird.

## Claims

1. Apparatus (1) for preparing acrylic acid, comprising
- a first reactor (2) and
- at least one further reactor (3),
-- wherein at least the first reactor (2) is a shell-and-tube reactor having a plurality of tubes (4) which comprise a catalyst which catalyses the synthesis of acrolein,
-- wherein the tubes (4) open into a collection space (6) which is fluidically connectable via an outflow region (7) to the at least one further reactor (3),
-- wherein the outflow region (7) comprises a laminarizing means (8) which effects laminarization of a flow profile of a gas flowing through the outflow region (8) and
-- wherein the laminarizing means (8) projects into the outflow region (7).

2. Apparatus (1) according to Claim 1, wherein the laminarizing means (8) tapers in the direction of the outflow region (7).

3. Apparatus (1) according to either of the preceding claims, wherein the laminarizing means (8) comprises a truncated cone.

4. Apparatus according to Claim 2 or 3, wherein the laminarizing means (8) comprises a cone.

5. Apparatus according to Claim 3 or 4, wherein the cone or truncated cone has a cone angle (13) in the range from 15 to 60°.

6. Apparatus according to Claim 5, wherein the ratio of the height of the cone or truncated cone (H) :
diameter of the cone or truncated cone on the side (D) facing away from the at least one further reactor is in the range from 3 : 1 to 1 : 3.

7. Process for preparing acrylic acid, wherein
- acrolein is produced in a first stage by gas-phase oxidation of propene in a first reactor (2)
- and acrolein is converted into acrylic acid in at least one second stage in at least one further reactor (3),
-- wherein the first reactor (2) is a shell-and-tube reactor having a plurality of tubes (4) through which gas can flow and connected via an outflow region (7) to at least one further reactor (3),
-- wherein a gas flow in the outflow region (7) is laminarized by means of a laminarizing means,
wherein the process is carried out in an apparatus (1) according to any of Claims 1 to 6.

## Revendications

1. Dispositif (1) pour la fabrication d'acide acrylique, comprenant :
- un premier réacteur (2) et
- au moins un autre réacteur (3),
-- au moins le premier réacteur (2) étant un réacteur à faisceau de tubes contenant une pluralité de tubes (4), qui comprennent un catalyseur qui catalyse la synthèse d'acroléine,
-- les tubes (4) débouchant dans une chambre de collecte (6), qui peut être raccordée fluidiquement par le biais d'une zone de sortie (7) avec ledit au moins un autre réacteur (3),
-- la zone de sortie (7) comprenant un agent de laminarisation (8), qui provoque une laminarisation d'un profil d'écoulement d'un gaz s'écoulant dans la zone de sortie (8), et
-- l'agent de laminarisation (8) avançant dans la zone de sortie (7).

2. Dispositif (1) selon la revendication 1, dans lequel l'agent de laminarisation (8) rétrécit dans la direction de la zone de sortie (7).

3. Dispositif (1) selon l'une quelconque des revendications précédentes, dans lequel l'agent de laminarisation (8) comprend un tronc de cône.

4. Dispositif selon la revendication 2 ou 3, dans lequel l'agent de laminarisation (8) comprend un cône.

5. Dispositif selon la revendication 3 ou 4, dans lequel le cône ou le tronc de cône présente un angle de cône (13) dans la plage allant de 15 à 60°.

6. Dispositif selon la revendication 5, dans lequel le rapport entre la hauteur du cône ou du tronc de cône (H):diamètre du cône ou du tronc de cône sur le côté (D) opposé audit au moins un autre réacteur se situe dans une plage allant de 3:1 à 1:3.

7. Procédé de fabrication d'acide acrylique, selon lequel
- lors d'une première étape, de l'acroléine est formée par oxydation en phase gazeuse de propène dans un premier réacteur (2),
- et lors d'au moins une deuxième étape dans au moins un autre réacteur (3), l'acroléine est transformée en acide acrylique,
-- le premier réacteur (2) étant un réacteur à faisceau de tubes contenant une pluralité de tubes (4) pouvant être traversés par un gaz, qui est raccordé par le biais d'une zone de sortie (7) avec au moins un autre réacteur (3),
-- un courant gazeux dans la zone de sortie (7) étant laminarisé au moyen d'un agent de laminarisation, le procédé étant réalisé dans un dispositif (1) selon l'une quelconque des revendications 1 à 6.
